# EUROPEAN PATENT SPECIFICATION

(11) **EP 3 644 949 B1**
(45) Date of publication and mention of the grant of the patent: **20.10.2021**
(21) Application number: 18734225.8
(22) Date of filing: 27.06.2018
(51) Int. Cl.: A61K 8/44, A61K 8/46, A61Q 5/02, A61K 8/73, A61Q 19/10, A61K 8/02

(54) **SOLID CLEANING COSMETIC COMPOSITION**
FESTE REINIGENDE KOSMETISCHE ZUSAMMENSETZUNG
COMPOSITION COSMÉTIQUE DE NETTOYAGE SOLIDE

(30) Priority: 30.06.2017 FR 1756219
(43) Date of publication of application: 06.05.2020
(73) Proprietor: L'Oreal, 75008 Paris (FR)
(72) Inventor: LAVABLE, Nadine, 93400 Saint Ouen (FR); MOYSAN, Pascale, 92110 Clichy (FR)
(74) Representative: Lavoix
(86) International application number: PCT/EP2018/067259
(87) International publication number: WO 2019/002374

(56) References cited:
- FR-A1- 3 030 269
- US-A1- 2008 152 711

## Description

This invention relates to a solid anhydrous cosmetic composition and a method of using said composition.

It is known that compositions in the form of cream, gels and liquid formulas can be used in the field of skin hygiene and/or cleaning products. Such formulas are usually packaged in flasks or other packagings that occupy a certain volume, and therefore have significant dimensions. Furthermore, due their liquid or semi-liquid texture, these compositions can partly escape from their packaging, particularly during transport, that causes leaks.

One alternative to this type of composition lies in the use of soap bars. They are in solid form and they have a small volume. However, once again, these formulas are not easy to transport once they have been used because they are wet. FR3030269A1 discloses in ex. 5 a solid anhydrous composition containing cocoyl isethionate.

Therefore there is a need for compositions, particularly hygiene and/or cleaning compositions that are easy to apply, easy to transport, lightweight and with no risk of leak. Such compositions must also require the use of a minimum quantity of water.

This invention responds to this need. It discloses a solid anhydrous solution that has a small volume, uses a small quantity of water and has no risk of leaks. In particular, such a composition is in the form of a tablet. It is easy to transport, lightweight and can be used to make a well-measured amount of product for the consumer.

Therefore the purpose of this invention is a solid anhydrous composition, preferably a cosmetic composition, comprising:
- at least 70% by weight of microcrystalline cellulose as a proportion of the total weight of the composition;
- at least one surfactant chosen from among isethionic derivatives and their cosmetically acceptable salts; and
- at least one surfactant chosen from among acyl glutamic acids and their cosmetically acceptable salts.
Optionally, the composition according to the invention comprises at least one lubricant.

This invention also relates to a method of preparing such a composition, including a mixture of microcrystalline cellulose with a surfactant chosen from among isethionic acid derivatives and their cosmetically acceptable salts, at least one surfactant chosen from among acyl glutamic acid derivatives and their cosmetically acceptable salts, and possibly at least one lubricant; then compression of the mix obtained.

Another purpose of this invention is a method of cleaning keratin materials (cosmetic process), preferably the skin and/or the hair, in which the solid anhydrous composition according to the invention is brought into contact with keratin materials, then rinsing, particularly with water.

In this description, the expression "at least" is equivalent to the expression "one or several" and can be substituted for it.

In this description, the expression "included between" is equivalent to the expression "from ... to" and can be substituted for it.

"Keratin materials" means the skin, the mucous membranes and/or skin appendages, and preferably the skin and/or hair.

The composition according to the invention is solid. "Solid" means a composition that does not flow under its own weight. The composition according to the invention may be in any solid galenic form, and particularly in the form of a tablet, lozenge, pellet or granule. Preferably, the composition according to the invention is in the form of tablet.

The composition according to the invention is anhydrous. "Anhydrous" means that the composition contains less than 5% by weight of water relative to the total weight of the composition, preferably less than 3%, more preferably less than 1% and ideally contains no water at all

During use, the anhydrous solution according to the invention typically dissolves rapidly in contact with water. Preferably, it forms a foam. It thus forms a solid hygiene product that is practical and easy to transport, with no risk of leak and with a small volume.

As demonstrated in the examples, the composition according to the invention has an innovative galenic form, and the different ingredients play an important role in this galenic form.

Preferably, the composition according to the invention contains no bicarbonate, and particularly no sodium bicarbonate. The solid anhydrous composition according to the invention dissolves in contact with water and can foam, but without effervescence.

### Microcrystalline cellulose

Compositions conforming with the invention comprise at least 70% by weight of microcrystalline cellulose as a proportion of the total weight of the composition.

The microcrystalline cellulose is generally prepared from wood pulp: said pump is cut into small particles that are chemically hydrolyzed, particularly with an inorganic acid, and a filtration and drying step then takes place.

The microcrystalline cellulose may particularly be that marketed by FMC Biopolymers under the name Avicel PH 102 or Avicel PH 105.

Preferably, the particle size of the microcrystalline cellulose is defined by a median diameter D50 ranging from 80 to 140 µm. It is preferably that marketed by FMC Biopolymers under the name Avicel PH 102.

Preferably, the content of microcrystalline cellulose is between 70% and 95% by weight, particularly from 72% to 90% by weight, and more preferably from 75% to 85% by weight, with respect to the weight of the composition.

### Isethionate surfactant

Compositions according to the invention comprise at least one surfactant chosen from among isethionic acid derivatives and their cosmetically acceptable salts. This surfactant is also called an "isethionate surfactant" in this application.

The salt or derivative of isethionic acid may for example be chosen among acyl isethionic acids, their salts (isethionates) and mixtures thereof. Preferably, it is chosen from among acyl isethionic acid salts for which the hydrocarbon chain R of the acyl group RC=O is linear or branched, saturated or unsaturated, and comprises from 8 to 30 carbon atoms, and preferably from 11 to 20 carbon atoms. The acyl group can be in particular chosen from the lauroyl, myristoyl, palmitoyl, stearoyl, olivoyl, cocoyl, oleoyl groups, and mixtures thereof.

"Cosmetically acceptable salts" means that the hydrogen atom in the SO3H group of the isethionic acid is replaced by a cation X, for example chosen from among alkali metal ions such as Na, Li, K, preferably Na or K; ions of alkali earth metals such as Mg; the ammonium groups; and mixtures thereof.

In particular, mention may be made of the formula (I):

R-C(=O)-C(R2)(R3)-C(R4)(R5)-SO3X (I)

in which R and X have the same meaning as above, and X may also be H⁺,
and R2, R3 and R4 and R5 are independently a hydrogen atom, or a linear or branched alkyl group, preferably linear, comprising from 1 to 4 carbon atoms.

For example, mention may be made of compounds with the INCI name sodium cocoyl isethionate, for example products marketed with the name Hostapon SCI and Hostapon STCI by Clariant, and particularly the following compounds: Hostapon SCI-78 C, Hostapon SCI-85 C, Hostapon SCI-78 P, Hostapon STCI-85 C, Hostapon STCI-85 G or Hostapon STCI-85 P; also compounds marketed under the Elfan name by Akzo, particularly such as Elfan AT 84 G, Elfan AT 84 or Elfan AT 90 G.

Mention may also be made of ammonium cocoyl isethionate, for example that marketed by BASF under the name Jordapon ACI-30.

For example, mention may be made of commercial mixtures containing sodium cocoyl isethionate like mixtures marketed by Clariant under the name Hostapon SCI-65C, Hostapon SCI-40 L and Hostapon STCI-65 C, or Biobase SMC by Tri-K industries, and Jordapon CI 65 by BASF.

Preferably, the isethionate surfactant is sodium cocoyl isethionate.

Preferably, the content of isethionate surfactant is between 2% and 20% by weight of the total weight of the composition, preferably between 2.5% and 15% by weight, preferably between 3% and 10% by weight.

### Glutamate surfactant

The composition according to the invention comprises at least one surfactant chosen from among acyl glutamic acids and their cosmetically acceptable salts. This surfactant is also called a "glutamate surfactant" in this application.

Preferably, a composition according to the invention comprises at least one acyl glutamic acid with at least one C12-C22 acyl chain, its salt (glutamate), or mixtures thereof. In particular, the glutamate surfactant is chosen from among acyl glutamic acids with a C12-C22 acyl chain, alkali metal salts such as Na, Li, K, preferably Na or K, alkali earth metal salts such as Mg, ammonium salts of said acids and mixtures thereof.

More preferably, the acyl glutamic acid (INCI name: acyl glutamic acid), its salts (glutamates) and mixtures thereof, are chosen from among lauroyl glutamic, myristoyl glutamic, palmitoyl glutamic, stearoyl glutamic, behenoyl glutamic, olivoyl glutamic, cocoyl glutamic acids and alkali metal salts such as Na, Li, K, preferably Na or K, alkali earth metal salts such as Mg, ammonium salts of said acids and mixtures thereof.

In particular, mention can be made of compounds with the INCI name lauroyl glutamic acid (particularly sodium lauroyl glutamic acid), cocoyl glutamic acid, sodium stearoyl glutamate, potassium lauroyl glutamate, potassium cocoyl glutamate, sodium olivoyl glutamate, and mixtures thereof. Such compounds are marketed under the name AMISOFT by AJINOMOTO and particularly references Amisoft CA, Amisoft LA, Amisoft HS 11 PF, Amisoft MK-11, Amisoft LK-11, Amisoft CK-11, or marketed by Keminova Italiana SRL.

Acyl glutamic acid salts include disodium hydrogenated tallow glutamate as marketed under reference Amisoft HS5 21 by AJINOMOTO. Mention can also be made of commercial mixtures of surfactants comprising at least one acyl glutamic salt, for example a mixture of acyl glutamate salts such as Amisoft LS-22 marketed by AJINOMOTO.

According to one particular embodiment, the mono-sodium salt of n-stearoyl-L-glutamic acid is used, and most particularly that marketed by AJINOMOTO as reference Amisoft HS 11 (INCI name: sodium stearoyl glutamate), or the mono-sodium salt of n-lauroyl-L-glutamic acid is used, and most particularly that marketed by AJINOMOTO as reference Amisoft LS 11 (INCI name: sodium lauroyl glutamate).

Preferably, the content of glutamate surfactant is between 0.1% and 30% by weight of the total weight of the composition, preferably between 1% and 25% by weight, preferably between 5% and 15% by weight.

Preferably, the total content of surfactant is between 5% and 35% by weight of the total weight of the composition, preferably between 10% and 30% by weight.

### Lubricant

The composition according to the invention can also comprise at least one lubricant. Such a lubricant can in particular play a role as anti-caking compound.

Among lubricants that can be used in compositions according to the invention, mention can be made of silica, particularly anhydrous colloidal silica, kaolin, starch, mica, talc, sericite, polyamide (Nylon®), poly-p-alanine and polyethylene powders, tetrafluoroethylene polymer powders (Teflon®), acrylate and dimethicone copolymers, sodium carbonate, precipitated calcium carbonate, magnesium carbonate and hydrocarbonate, stearic acid, polyethylene glycol, metal soaps derived from carboxylic organic acids with 8 to 22 carbon atoms, preferably 12 to 18 carbon atoms, for example zinc, magnesium or lithium stearate, zinc laurate and magnesium myristate and mixtures thereof.

Preferably, the lubricant is a metal soap derived from carboxylic organic acid with 8 to 22 carbon atoms, preferably 12 to 18 carbon atoms, for example zinc, magnesium or lithium stearate, zinc laurate and magnesium myristate. Preferably, the lubricant is magnesium stearate.

A composition implemented according to the invention may comprise one or more lubricants with a content ranging from 0.1% to 15% by weight, particularly from 1 to 10% by weight, preferably from 2 to 5% with respect to the weight of the composition.

Preferably, the composition according to the invention comprises:
- at least 70% by weight of microcrystalline cellulose as a proportion of the total weight of the composition;
- at least one isethionate surfactant;
- at least one glutamate surfactant; and
- possibly, at least one lubricant.

Preferably, the composition according to the invention comprises, preferably consists of, the following, by weight as a ratio of the total weight of the composition:
- from 70% to 95% of microcrystalline cellulose;
- from 2 to 10% of at least one isethionate surfactant;
- from 5% to 20% of at least one glutamate surfactant; and
- possibly from 1% to 10% of at least one lubricant.

### Binding agents

According to one particular embodiment of the invention, the composition comprises at least one liquid binding agent chosen from among the polyols such as glycerol, 1,3-propanediol, propylene glycol, butylene glycol, hexylene glycol, polyethylene glycols such as PEG-8 or dipropylene glycol, or preferably a solid binder.
In the framework of the invention, the solid binding agent(s) may be chosen from among:
- sugars such as mannitol, maltodextrin, sorbitol, xylitol, sucrose, glucose,
- cellulose derivatives,
- polyvinylpyrrolidone (PVP),
- starches,
- gums such as gum arabic, xanthan gum or carrageenans,
- and mixtures thereof.

Preferably, the composition according to this invention comprises at least one binder chosen from among starches, PVP, sugars (such as mannitol, sorbitol or sucrose), cellulose derivatives (such as carboxymethylcellulose) and gums (such as xanthan gum or gum arabic).

The composition according to the invention may comprise different additives, such as dyes, active constituents and particularly additives that are hydrophobic or sensitive to water (for example retinol or anti-dandruff agents), and/or preservatives. Preferably, the composition according to the invention has no preservative.

Another purpose of this invention is a method of preparing such a composition, including the mix of microcrystalline cellulose with at least one surfactant chosen from among isothionic acid derivatives and their cosmetically acceptable salts, at least one surfactant chosen from among acyl glutamic acid derivatives and their cosmetically acceptable salts, and possibly at least one lubricant; then compression of the mix obtained.

In particular, the mixing step may be performed using a high shear mixer granulator such as those made by Lodige, Backer-Perkins, Bosch, Amixon, Promixon or Glatt brands; a plough mixer such as those made by Loedige, Zeppelin or Promixon; a screw mixer such as those sold by Amixon, Parimix or Hosokawa; or a tumbler mixer such as those from Bachilla or Servolift. Preferably, a high shear mixer granulator or a plough mixer will be used such as those made by Loedige or Backer-Perkins.

In particular, it is done at ambient temperature.

The compression step of the mix obtained can be done using a press, particularly a tablet press.

Such a process is illustrated in the examples.

Finally, one purpose of this invention is a method of cleaning keratin materials, preferably the skin and/or the hair, in which the solid anhydrous composition according to the invention is brought into contact with keratin materials, then rinsing, particularly with water.

The invention can be better understood with reference to the non-limitative examples given below that constitute preferred methods of implementing the method according to the invention.

Unless mentioned otherwise, quantities in the following examples are expressed as a percent by weight.

### Example 1:

### 1.1. The following compositions are prepared:

| **Raw materials** | **Composition according to the invention** | **Comparative composition A** | **Comparative composition B** |
|---|---|---|---|
| Sodium Lauroyl Glutamate (Amisoft LS 11) | **10.00%** | - | - |
| Sodium Cocoyl Isethionate (Hostapon SCI) | **5.00%** | - | **20.00%** |
| Sodium Lauryl Sulfate (Texapon) | - | **15.00%** | - |
| Microcrystalline cellulose (Avicel PH 102) | **81.00%** | **60.00%** | **60.00%** |
| Powder magnesium stearate (DUB SMG) | **4.00%** | **10.00%** | - |
| Crosslinked sodium carboxymethyl starch (Sodium Starch Glycolate, Glycolys) | - | **15.00%** | - |
| Sodium carboxymethyl starch purified to 99.5% (BLANOSE CMC 7H4XF) | - | - | **15.00%** |
| Gum arabic (SPRAYGUM SC10) | - | - | **5.00%** |

The different ingredients in each composition are mixed in a Backer 1L mixer granulator, with the following operating conditions:

| **Composition according to the invention** | | | | | | | |
|---|---|---|---|---|---|---|---|
| **Actions** | **Tank bottom vane (rpm)** | **Decaker (rpm)** | **Duration** | **T°C of the double wall** | **Vp vane (m/s)** | **Vp decaker (m/s)** | **Observations** |
| Powder mix | 705 | 660 | 1min30 | 20°C | 4.17 | 1.07 | A fine and homogeneous powder is obtained, no caking, the powder flows well. |

| **Comparative composition A** | | | | | | | |
|---|---|---|---|---|---|---|---|
| **Actions** | **Tank bottom vane (rpm)** | **Decaker (rpm)** | **Duration** | **T°C double wall** | **Vp vane (m/s)** | **Vp decaker (m/s)** | **Observations** |
| Powder mix | 709 | 648 | 1min30 | 20°C | 4.27 | 0.92 | A fine and homogeneous powder is obtained, no caking, the powder flows **well but it sticks to the metal.** |

| **Comparative composition B** | | | | | | | |
|---|---|---|---|---|---|---|---|
| **Actions** | **Tank bottom vane (rpm)** | **Decaker (rpm)** | **Duration** | **T°C of the double wall** | **Vp vane (m/s)** | **Vp decaker (m/s)** | **Observations** |
| Powder mix | 705 | 663 | 1min30 | 20°C | 4.24 | 0.94 | Carboxymethylcellulose is electrostatic (it fixes to the metal walls) and gum arabic is sticky. **There are clumps in** t**he bulk material at the end of mixing. Stirring is prolonged** |
| Powder mix | 722 | 652 | 1 min | 20°C | 4.34 | 0.92 | Modified parameters: A fine and homogeneous powder is obtained, no caking. |

The above tables show that at the end of mixing, only the composition according to the invention produces a fine homogeneous powder with no caking and that flows well.

The compositions are then compressed using a Dott Bonapace CPR-6 tablet press with an identical punch lowering distance and force, the compression rate also being identical. The height of the bottom punch is adjusted by turning the adjustment screw by 8 half-turns, starting from the lowest possible level. Lowering of the top punch is set to level 5.3. The automatic speed is set to 3, namely compression of 750 tablets/hour.

The compression conditions are as follows:

| **Compression conditions** | | |
|---|---|---|
| **Formulas** | **% Humidity** | **T°C in the zone** |
| **Composition according to the invention** | **30.6%** | **20.5°C** |
| **Comparative composition A** | **30.9%** | **20.3°C** |
| **Comparative composition B** | **30.6%** | **20.2°C** |

### 1.2. Evaluation of the prepared compositions

One tablet of each composition is taken in the hand.

Then in contact with water:
- the composition according to the invention (tablet) starts to disintegrate. It can then easily be crushed by friction; all that remains to be done is to rub your hands to create a foam;
- comparative composition A (tablet) disintegrates a little. After applying friction in the hand several times, the tablet does not dissolve; and
- comparative composition B (tablet) does not disintegrate. After applying friction in the hand several times, the tablet does not dissolve at all; and no foam is formed.

Therefore the composition according to the invention is the only composition that disintegrates in contact with water.

### Example 2: Evaluation of the quantity of microcrystalline cellulose

### 2.1. The following compositions are prepared:

| **Raw materials** | **Composition according to the invention** | **Alternative composition according to the invention** | **Comparative composition C** |
|---|---|---|---|
| Sodium Lauroyl Glutamate (Amisoft LS 11) | 10.00% | 10.00% | 10.00% |
| Sodium Cocoyl Isethionate (Hostapon SCI) | 5.00% | 10.00% | 20.00% |
| Microcrystalline cellulose (Avicel PH 102) | **81.00%** | **76.00%** | **66.00%** |
| Magnesium stearate (DUB SMG) | 4.00% | 4.00% | 4.00% |

The different ingredients in each composition are mixed in a Backer 1L mixer granulator, with the operating conditions used in example 1:
The compositions are then compressed using a Dott Bonapace CPR-6 tablet press with 15 mm diameter punches. The height of the bottom punch is adjusted by turning the adjustment screw by 8 half-turns, starting from the lowest possible level. Lowering of the top punch is set to level 3 and the automatic speed is set to 10, namely 2500 tablets/hour.

The following densities are obtained:

| **Composition according to the invention** | **Alternative composition according to the invention** | **Comparative composition C** |
|---|---|---|
| 0.872 g/cm3 | 0.863 g/cm3 | 0.846 g/cm3 |

### 2.2. Evaluation of the prepared compositions

One tablet of each composition is taken in the hand.

Then in contact with water:
- the composition according to the invention (tablet) starts to disintegrate. After applying friction in the hand several times, the tablet dissolves easily;
- the alternative composition according to the invention (tablet) starts to disintegrate. After applying friction in the hand several times, the tablet dissolves easily; and
- comparative composition C (tablet) disintegrates very little. It is very difficult to crumble; a residue remains.

Therefore the only compositions that disintegrate in contact with water are the two compositions according to the invention (with a microcrystalline cellulose content of 81% and 76% by weight).

### Example 3: Evaluation of the nature of surfactants

### 3.1. The following compositions are prepared:

| **Raw materials** | **Composition according to the invention** | **Comparative composition D** | **Comparative composition E** |
|---|---|---|---|
| Sodium Lauroyl Glutamate (Amisoft LS 11) | 10.00% | - | - |
| Sodium Cocoyl Isethionate (Hostapon SCI) | 5.00% | - | 5.00% |
| Sodium Lauroyl Sarcosinate (GALSOFT NALS (P)) | - | 15.00% | 10.00% |
| Microcrystalline cellulose (Avicel PH 102) | 81.00% | 81.00% | 81.00% |
| Magnesium stearate (DUB SMG) | 4.00% | 4.00% | 4.00% |

The different ingredients in each composition are mixed in a Backer 1L mixer granulator, with the operating conditions used in example 1.

### 3.2. Evaluation of the prepared compositions

One tablet of each composition is taken in the hand.
Then in contact with water:
- the composition according to the invention (tablet) starts to disintegrate. After applying friction in the hand several times, the tablet dissolves easily;
- the comparative composition D (tablet) disintegrates quickly in contact with water. From a cosmetic point of view, the sarcosinate is not as rough as the glutamate. **However, it is friable;** and
- the comparative composition E (tablet) disintegrates It is very difficult to crumble; a residue remains.

Therefore the composition according to the invention, that includes the combination of an isethionate surfactant and a glutamate surfactant is the only one that disintegrates in contact with water.

### Example 4: Evaluation of the nature of disintegrating agents

### 4.1. The following compositions are prepared:

| Raw materials | **Composition according to the invention** | **Comparative composition F** | **Comparative composition G** | **Comparative composition H** |
|---|---|---|---|---|
| Sodium Lauroyl Glutamate (Amisoft LS 11) | **10.00%** | **10.00%** | **10.00%** | **10.00%** |
| Sodium Cocoyl Isethionate (Hostapon SCI) | **5.00%** | **5.00%** | **5.00%** | **5.00%** |
| Magnesium stearate (DUB SMG) | **4.00%** | **4.00%** | **4.00%** | **4.00%** |
| Microcrystalline cellulose (Avicel PH 102) | **81.00%** | - | - | - |
| Carbonate | - | **68.00%** | - | - |
| Citric acid | - | **13.00%** | - | - |
| Crosslinked sodium carboxymethyl starch (Sodium Starch Glycolate, Glycolys) | - | - | **81.00%** | - |
| Crosslinked polyacrylate micro spheres (ref com) | - | - | - | **81.00%** |

The different ingredients in each composition are mixed in a Backer 1L mixer granulator, with the operating conditions used in example 1.

### 4.2. Evaluation of the prepared compositions

| **Composition according to the invention** | **Comparative composition F** | **Comparative composition G** | **Comparative composition H** |
|---|---|---|---|
| The pellets form well | The product sticks to the punches. There are some blisters on the tablets. Degassing occurs when the flask containing the pellets is opened! Effervescence has begun. At the time of compression, the state of the citric acid changes. | The powder is very fine, it has very good flowability but does not compress well. There is a "capping" phenomenon. The tablet is fragile and breaks when it drops into the tank (even with foam to dampen it). | The powder does not compress at all, there is no cohesion, the tests are stopped. The tablet is crushed with one finger. |

### Conclusion

The above results show that at least 70% of microcrystalline cellulose is necessary for fast crumbling. Use of the isethionate surfactant such as sodium cocoyl isethionate as the cohesion agent is more appropriate than gum arabic because the tablet crumbles more quickly forming a foam.

The anhydrous solid compositions according to the invention dissolve quickly in the hand and are capable of foaming. Therefore they form good cleaning products. Furthermore, they can be obtained by direct compression of the mixed ingredients.

## Claims

1. Solid anhydrous composition, preferably cosmetic, containing:
- at least 70% by weight of microcrystalline cellulose as a proportion of the total weight of the composition;
- at least one surfactant chosen from among isethionic derivatives and their cosmetically acceptable salts;
- at least one surfactant chosen from among acyl glutamic acids and their cosmetically acceptable salts,
and wherein the anhydrous composition contains less than 5% by weight of water relative to the total weight of the composition.

2. Composition according to claim 1, in which the surfactant is chosen from among derivatives of isethionic acid and their cosmetically acceptable salts and is chosen from among acyl isethionic acid salts for which the hydrocarbon chain R of the acyl group RC=O is linear or branched, saturated or unsaturated, and comprises from 8 to 30 carbon atoms, and preferably from 11 to 20 carbon atoms.

3. Composition according to claim 1 or 2, in which the surfactant chosen from among derivatives of isethionic acid and their cosmetically acceptable salts is a compound with formula (I):
R-C(=O)-C(R2)(R3)-C(R4)(R5)-SO3X (I)
in which:
R represents a linear or branched, saturated or unsaturated hydrocarbon chain, and comprises from 8 to 30 carbon atoms, preferably from 11 to 20 carbon atoms;
X is chosen from among H⁺, ions of alkali metals such as Na, Li, K, preferably Na or K; ions of alkali earth metals such as Mg; ammonium groups; and mixtures thereof; and
R2, R3 and R4 and R5 independently represent a hydrogen atom, or a linear or branched alkyl group, preferably linear, comprising from 1 to 4 carbon atoms.

4. Composition according to one of the previous claims, in which the content of surfactant chosen from among derivatives of isethionic acid and their cosmetically acceptable salts is between 2% and 20% by weight of the total weight of the composition, preferably between 2.5% and 15% by weight, preferably between 3% and 10% by weight

5. Composition according to one of the previous claims, in which the surfactant chosen from among acyl glutamic acids and their cosmetically acceptable salts is chosen from among acyl glutamic acids with a C12-C22 acyl chain, alkali metal salts such as Na, Li, K, alkali earth metal salts such as Mg, ammonium salts of said acids and mixtures thereof.

6. Composition according to one of the previous claims, in which the content of surfactant chosen from among acyl glutamic acids and their cosmetically acceptable salts is between 0.1% and 30% by weight of the total weight of the composition, preferably between 1% and 25% by weight, preferably between 5% and 15% by weight

7. Composition according to one of the previous claims, in which the content of microcrystalline cellulose is between 70% and 95% by weight, particularly from 72% to 90% by weight, and more preferably from 75% to 85% by weight, with respect to the weight of the composition.

8. Composition according to one of the previous claims, that also comprises at least one lubricant.

9. Composition according to claim 8, in which the lubricant is chosen from among silica, particularly anhydrous colloidal silica, kaolin, starch, mica, talc, sericite, polyamide, poly-p-alanine and polyethylene powders, tetrafluoroethylene polymer powders, acrylate and dimethicone copolymers, sodium carbonate, precipitated calcium carbonate, magnesium carbonate and hydrocarbonate, stearic acid, polyethylene glycol, metal soaps derived from carboxylic organic acids with 8 to 22 carbon atoms, preferably 12 to 18 carbon atoms, and mixtures thereof.

10. Composition according to either claim 8 or 9, in which the lubricant is present with a content of ranging from 0.1% to 15% by weight, particularly from 1% to 10% by weight and better from 2% to 5% by weight with respect to the total weight of the composition.

11. Composition according to one of the previous claims, that comprises.
- at least 70% by weight of microcrystalline cellulose as a proportion of the total weight of the composition;
- at least one surfactant chosen from among isethionic derivatives and their cosmetically acceptable salts;
- at least one surfactant chosen from among acyl glutamic acids and their cosmetically acceptable salts; and
- possibly, at least one lubricant.

12. Composition according to one of the previous claims, comprising the following, by weight as a ratio of the total weight of the composition:
- from 70% to 90% of microcrystalline cellulose;
- from 2 to 10% of at least one surfactant chosen from among isethionic derivatives and their cosmetically acceptable salts;
- from 5% to 20% of at least one surfactant chosen from among acyl glutamic acids and their cosmetically acceptable salts; and
- possibly from 1% to 10% of at least one lubricant.

13. Composition according to one of the previous claims, that also comprises at least one liquid binding agent chosen from among the polyols such as glycerol, 1,3-propanediol, propylene glycol, butylene glycol, hexylene glycol, polyethylene glycols such as PEG-8 or dipropylene glycol, or preferably a solid binder, chosen from among:
- sugars such as mannitol, maltodextrin, sorbitol, xylitol, sucrose, glucose,
- cellulose derivatives,
- polyvinylpyrrolidone (PVP),
- starches,
- gums such as gum arabic, xanthan gum or carrageenans,
- and mixtures thereof.

14. Composition according to one of the previous claims, that is in the form of a tablet.

15. Method of preparing a composition according to one of the previous claims, comprising a mixture of microcrystalline cellulose with a surfactant chosen from among isethionic acid derivatives and their cosmetically acceptable salts, at least one surfactant chosen from among acyl glutamic acid derivatives and their cosmetically acceptable salts, and possibly at least one lubricant; then compression of the mix obtained.

16. Method of cleaning keratin materials, preferably the skin and/or the hair, in which the composition according to one of claims 1 to 14 is brought into contact with keratin materials, then rinsing, particularly with water.

## Patentansprüche

1. Feste, wasserfreie Zusammensetzung, vorzugsweise kosmetisch, aufweisend:
- mindestens 70 Gew.-% mikrokristalline Cellulose, als ein Anteil des Gesamtgewichts der Zusammensetzung;
- mindestens ein Tensid, ausgewählt unter Isethionderivaten und ihren kosmetisch verträglichen Salzen;
- mindestens ein Tensid, ausgewählt unter Acylglutaminsäuren und ihren kosmetisch verträglichen Salzen,
und wobei die wasserfreie Zusammensetzung weniger als 5 Gew.-% Wasser enthält, bezogen auf das Gesamtgewicht der Zusammensetzung.

2. Zusammensetzung gemäß Anspruch 1, bei der das Tensid ausgewählt ist unter Derivaten der Isethionsäure und ihren kosmetisch verträglichen Salzen und ausgewählt ist unter Acylisethionsäuresalzen, bei denen die Kohlenwasserstoffkette R der Acylgruppe RC=O linear oder verzweigt, gesättigt oder ungesättigt, ist und 8 bis 30 Kohlenstoffatome aufweist, vorzugsweise 11 bis 20 Kohlenstoffatome.

3. Zusammensetzung gemäß Anspruch 1 oder 2, bei der das Tensid, das ausgewählt ist unter Derivaten der Isethionsäure und ihren kosmetisch verträglichen Salzen, eine Verbindung der Formel (I) ist:
R-C(=O)-C(R2)(R3)-C(R4)(R5)-SO3X (I)
bei der:
R eine lineare oder verzweigte, gesättigte oder ungesättigte, Kohlenwasserstoffkette darstellt und 8 bis 30 Kohlenstoffatome aufweist, vorzugsweise 11 bis 20 Kohlenstoffatome;
X ausgewählt ist unter H⁺, Ionen von Alkalimetallen wie Na, Li, K, vorzugsweise Na oder K; Ionen von Erdalkalimetallen wie Mg; Ammoniumgruppen; und deren Mischungen; und
R2, R3 und R4 und R5, unabhängig voneinander, ein Wasserstoffatom oder eine lineare oder verzweigte, vorzugsweise lineare, Alkylgruppe mit 1 bis 4 Kohlenstoffatomen darstellen.

4. Zusammensetzung gemäß einem der vorhergehenden Ansprüche, bei der der Gehalt an Tensid, ausgewählt unter Derivaten von Isethionsäure und ihren kosmetisch verträglichen Salzen, zwischen 2 und 20 Gew.-% des Gesamtgewichts der Zusammensetzung liegt, vorzugsweise zwischen 2,5 und 15 Gew.-%, vorzugsweise zwischen 3 und 10 Gew.-%.

5. Zusammensetzung gemäß einem der vorhergehenden Ansprüche, bei der das Tensid, das ausgewählt ist unter Acylglutaminsäuren und ihren kosmetisch verträglichen Salzen, ausgewählt ist unter Acylglutaminsäuren mit einer C12-C22-Acylkette, Alkalimetallsalzen wie Na, Li, K, Erdalkalimetallsalzen wie Mg, Ammoniumsalzen der besagten Säuren und deren Mischungen.

6. Zusammensetzung gemäß einem der vorhergehenden Ansprüche, bei der der Gehalt an Tensid, ausgewählt unter Acylglutaminsäuren und ihren kosmetisch verträglichen Salzen, zwischen 0,1 und 30 Gew.-% liegt, bezogen auf das Gesamtgewicht der Zusammensetzung, vorzugsweise zwischen 1 und 25 Gew.-%, vorzugsweise zwischen 5 und 15 Gew.-%.

7. Zusammensetzung gemäß einem der vorhergehenden Ansprüche, bei der der Gehalt an mikrokristalliner Cellulose zwischen 70 und 95 Gew.-% liegt, bezogen auf das Gewicht der Zusammensetzung, insbesondere von 72 bis 90 Gew.-% und, weiter bevorzugt, von 75 bis 85 Gew.-%.

8. Zusammensetzung gemäß einem der vorhergehenden Ansprüche, die außerdem mindestens ein Gleitmittel enthält.

9. Zusammensetzung gemäß Anspruch 8, bei der das Gleitmittel ausgewählt ist unter Siliziumdioxid, insbesondere wasserfreiem kolloidalem Siliziumdioxid, Kaolin, Stärke, Glimmer, Talkum, Sericit, Polyamid, Poly-p-Alanin und Polyethylenpulvern, Tetrafluorethylen-Polymerpulvern, Acrylat und Dimethicon-Copolymeren, Natriumcarbonat, gefälltem Calciumcarbonat, Magnesiumcarbonat und Hydrocarbonat, Stearinsäure, Polyethylenglykol, Metallseifen, abgeleitet von organischen Carbonsäuren mit 8 bis 22 Kohlenstoffatomen, vorzugsweise 12 bis 18 Kohlenstoffatomen, und deren Mischungen.

10. Zusammensetzung gemäß entweder Anspruch 8 oder Anspruch 9, bei der das Gleitmittel mit einem Gehalt in einem Bereich von 0,1 bis 15 Gew.-% vorliegt, insbesondere von 1 bis 10 Gew.-% und, besser, von 2 bis 5 Gew.-%, bezogen auf das Gesamtgewicht der Zusammensetzung.

11. Zusammensetzung gemäß einem der vorhergehenden Ansprüche, die aufweist:
- mindestens 70 Gew.-% mikrokristalline Cellulose, als ein Anteil des Gesamtgewichts der Zusammensetzung;
- mindestens ein Tensid, ausgewählt unter Isethionderivaten und ihren kosmetisch verträglichen Salzen;
- mindestens ein Tensid, ausgewählt unter Acylglutaminsäuren und ihren kosmetisch verträglichen Salzen; und
- gegebenenfalls mindestens ein Gleitmittel.

12. Zusammensetzung gemäß einem der vorhergehenden Ansprüche, aufweisend das Folgende, in Gewicht, als ein Anteil des Gesamtgewichts der Zusammensetzung:
- 70 bis 90 % mikrokristalline Cellulose;
- 2 bis 10 % mindestens eines Tensids, ausgewählt unter Isethionderivaten und ihren kosmetisch verträglichen Salzen;
- 5 bis 20 % mindestens eines Tensids, ausgewählt unter Acylglutaminsäuren und ihren kosmetisch verträglichen Salzen; und
- gegebenenfalls 1 bis 10 % mindestens eines Gleitmittels.

13. Zusammensetzung gemäß einem der vorhergehenden Ansprüche, die außerdem enthält mindestens ein flüssiges Bindemittel, ausgewählt unter Polyolen, wie Glycerin, 1,3-Propandiol, Propylenglykol, Butylenglykol, Hexylenglykol, Polyethylenglykole wie PEG-8 oder Dipropylenglykol, oder, vorzugsweise, ein festes Bindemittel ausgewählt aus:
- Zuckern wie Mannit, Maltodextrin, Sorbit, Xylit, Saccharose, Glucose,
- Cellulosederivaten,
- Polyvinylpyrrolidon (PVP),
- Stärken,
- Gummis wie Gummi-Arabicum, Xanthan-Gummi oder Carrageen,
- und deren Mischungen.

14. Zusammensetzung gemäß einem der vorhergehenden Ansprüche, die in Form einer Tablette vorliegt.

15. Verfahren zur Herstellung einer Zusammensetzung gemäß einem der vorhergehenden Ansprüche, aufweisend eine Mischung aus mikrokristalliner Cellulose mit einem Tensid, ausgewählt unter Isethionderivaten und ihren kosmetisch verträglichen Salzen, mindestens einem Tensid, ausgewählt unter Acylglutaminsäurederivaten und ihren kosmetisch verträglichen Salzen und, gegebenenfalls, mindestens ein Gleitmittel; anschließendes Komprimieren der erhaltenen Mischung.

16. Verfahren zum Reinigen von Keratin-Materialien, vorzugsweise der Haut und/oder der Haare, bei dem die Zusammensetzung nach einem der Ansprüche 1 bis 14 mit Keratin-Materialien in Kontakt gebracht wird und anschließend, insbesondere mit Wasser, gespült wird.

## Revendications

1. Composition anhydre solide, de préférence cosmétique, comprenant :
- au moins 70% en poids par rapport au poids total de composition de cellulose microcristalline ;
- au moins un tensioactif choisi parmi les dérivés d'acide iséthionique et leurs sels cosmétiquement acceptables ;
- au moins un tensioactif choisi parmi les acides acyl glutamiques et leurs sels cosmétiquement acceptables,
et dans laquelle la composition anhydre contient moins de 5% en poids d'eau par rapport au poids total de composition.

2. Composition selon la revendication 1, dans laquelle le tensioactif est choisi parmi les dérivés d'acide iséthionique et leurs sels cosmétiquement acceptables et est choisi parmi les sels d'acide acyl iséthionique dont la chaîne hydrocarbonée R du groupement acyl RC=O est linéaire ou ramifiée, saturée ou insaturée, et comprend de 8 à 30 atomes de carbone, de préférence de 11 à 20 atomes de carbone.

3. Composition selon la revendication 1 ou 2, dans laquelle le tensioactif choisi parmi les dérivés d'acide iséthionique et leurs sels cosmétiquement acceptables est un composé de formule (I) :
R-C(=O)-C(R2)(R3)-C(R4)(R5)-SO3X (I)
dans laquelle :
R représente une chaîne hydrocarbonée linéaire ou ramifiée, saturée ou insaturée, et comprend de 8 à 30 atomes de carbone, de préférence de 11 à 20 atomes de carbone,
X est choisi parmi H⁺, les ions des métaux alcalins tels que Na, Li, K, de préférence Na ou K ; les ions des métaux alcalino-terreux tels que Mg ; les groupes ammonium ; et leurs mélanges ; et
R2, R3, R4 et R5 représentent indépendamment un atome d'hydrogène ou un groupe alkyle linéaire ou ramifié, de préférence linéaire, comprenant de 1 à 4 atomes de carbone.

4. Composition selon l'une des revendications précédentes, dans laquelle la teneur en tensioactif choisi parmi les dérivés d'acide iséthionique et leurs sels cosmétiquement acceptables est comprise entre 2% et 20% en poids par rapport au poids total de composition, de préférence entre 2,5% et 15% en poids, de préférence entre 3% et 10% en poids.

5. Composition selon l'une des revendications précédentes, dans laquelle le tensioactif choisi parmi les acides acyl glutamiques et leurs sels cosmétiquement acceptables est choisi parmi les acides acyl glutamiques ayant une chaîne acyle en C12-C22, les sels de métal alcalin tel que Na, Li, K, les sels de métaux alcalino-terreux tels que Mg, les sels d'ammonium desdits acides, et leurs mélanges.

6. Composition selon l'une des revendications précédentes, dans laquelle la teneur en tensioactif choisi parmi les acides acyl glutamiques et leurs sels cosmétiquement acceptables est comprise entre 0,1% et 30% en poids par rapport au poids total de composition, de préférence entre 1% et 25% en poids, de préférence entre 5% et 15% en poids.

7. Composition selon l'une des revendications précédentes, dans laquelle la teneur en cellulose microcristalline est comprise entre 70% et 95% en poids, préférentiellement de 72% à 90% en poids, plus préférentiellement de 75% à 85% en poids par rapport au poids total de la composition.

8. Composition selon l'une des revendications précédentes, qui comprend également au moins un lubrifiant.

9. Composition selon la revendication 8, dans laquelle le lubrifiant est choisi parmi la silice, notamment la silice colloïdale anhydre, le kaolin, l'amidon, le mica, le talc, la séricite, les poudres de polyamide, de poly-p-alanine et de polyéthylène, les poudres de polymères de tétrafluoroéthylène, les copolymères d'acrylate et de dimethicone, le carbonate de sodium, le carbonate de calcium précipité, le carbonate et l'hydro-carbonate de magnésium, l'acide stéarique, le polyéthylène glycol, les savons métalliques dérivés d'acides organiques carboxyliques ayant de 8 à 22 atomes de carbone, de préférence de 12 à 18 atomes de carbone, et leurs mélanges.

10. Composition selon l'une des revendications 8 ou 9, dans laquelle le lubrifiant est présent en une teneur allant de 0,1% à 15% en poids, en particulier de 1% à 10% en poids, de préférence de 2% à 5% en poids par rapport au poids de la composition.

11. Composition selon l'une des revendications précédentes, qui comprend :
- au moins 70% en poids par rapport au poids total de composition de cellulose microcristalline ;
- au moins un tensioactif choisi parmi les dérivés iséthioniques et leurs sels cosmétiquement acceptables ;
- au moins un tensioactif choisi parmi les acides acyl qlutamiques et leurs sels cosmétiquement acceptables ; et
- éventuellement au moins un lubrifiant.

12. Composition selon l'une des revendications précédentes, comprenant, en poids par rapport au poids total de la composition :
- de 70% à 90% de cellulose microcristalline ;
- de 2 à 10% d'au moins un tensioactif choisi parmi les dérivés iséthioniques et leurs sels cosmétiquement acceptables ;
- de 5% à 20% d'au moins un tensioactif choisi parmi les acides acyl qlutamiques et leurs sels cosmétiquement acceptables ; et
- éventuellement de 1% à 10% d'au moins un lubrifiant.

13. Composition selon l'une des revendications précédentes, qui comprend également au moins un agent liant liquide choisi parmi les polyols tels que le glycérol, le 1,3-propanediol, le propylène glycol, le butylène glycol, l'hexylène glycol, les polyéthylène glycols tels que le PEG-8 ou le dipropylène glycol, ou préférentiellement un liant solide choisi parmi :
- les sucres comme le mannitol, la maltodextrine, le sorbitol, le xylitol, le sucrose, le glucose,
- les dérivés de cellulose,
- la polyvinyl pyrrolidone (PVP),
- les amidons,
- les gommes, telle que la gomme arabique, la gomme de xanthane ou les carraghénanes,
- et leurs mélanges.

14. Composition selon l'une des revendications précédentes, qui se présente sous forme de comprimé.

15. Procédé de préparation d'une composition selon l'une des revendications précédentes, comprenant le mélange de la cellulose microcristalline avec au moins un tensioactif choisi parmi les dérivés d'acide iséthionique et leurs sels cosmétiquement acceptables, au moins un tensioactif choisi parmi les acides acyl glutamiques et leurs sels cosmétiquement acceptables, et éventuellement au moins un lubrifiant ; puis la compression du mélange obtenu.

16. Procédé de nettoyage des matières kératiniques, de préférence de la peau et/ou des cheveux, comprenant la mise en contact d'une composition selon l'une des revendications 1 à 14 avec les matières kératiniques, puis le rinçage, notamment à l'eau.
